# EUROPEAN PATENT APPLICATION

(11) **EP 1 253 141 A1**
(43) Date of publication of application: **30.10.2002**
(21) Application number: 01401097.9
(22) Date of filing: 27.04.2001
(51) Int. Cl.: C07D 209/86, A61K 31/403, A61P 35/00

(54) **Substituted carbazoles as tubulin polymerization inhibitors and their use for the treatment of cancer**

(71) Applicant: Aventis Pharma S.A., 92160 Antony (FR)
(72) Inventor: Caulfield, Thomas, 75016 Paris (FR); Cherrier, Marie-Pierre, 94200 Ivry sur Seine (FR); Combeau, Cécile, 92370 Chaville (FR); Mailliet, Patrick, 94120 Fontenay sous Bois (FR)
(74) Representative: Le Pennec, Magali

(57) **Abstract**

New alkoxyphenyl carbazolyl propan- or 2-propen-1-ol or 1-one compounds of structure (I) used as tubulin polymerization-inhibiting compounds as well as new pharmaceutical compositions containing them.

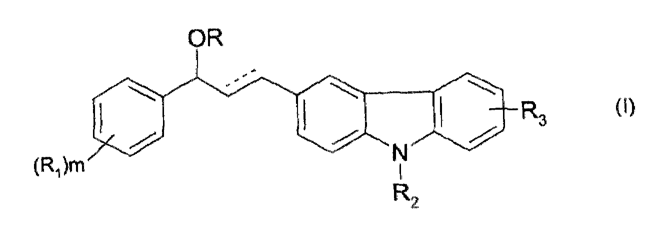

## Description

### Technical Field

The present invention relates to compounds useful for treating pathological states, which arise from or are exacerbated by cell proliferation, to pharmaceutical compositions comprising these compounds, and to methods of inhibiting cell proliferation in a mammal.

### Background of the Invention

Neoplastic diseases, characterized by the proliferation of cells, which are not subject to normal cell proliferating controls, are a major cause of death in humans and other mammals. Cancer chemotherapy has provided new and more effective drugs to treat these diseases and has also demonstrated that drugs, which disrupt microtubule synthesis, are effective in inhibiting the proliferation of neoplastic cells.

Microtubules play a key role in the regulation of cell architecture, metabolism and division. The microtubule system of eucaryotic cells comprises a dynamic assembly and disassembly matrix in which heterodimers of tubulin polymerize to form microtubules in both normal and neoplastic cells. Within neoplastic cells, tubulin is polymerized into microtubules, which form the mitotic spindle. The microtubules are then depolymerized when the mitotic spindle's use has been fulfilled. Agents, which disrupt the polymerization or depolymerization of microtubules in neoplastic cells, thereby inhibiting the proliferation of these cells, comprise some of the most effective cancer chemotherapeutic agents in use.

Because of the pivotal role played by cell proliferation, agents, which inhibit microtubule polymerization, have been the subjects of active current research for their clinical potential. But there is still a need for tubulin polymerization-inhibiting compounds with modified or improved profiles of activity.

### Summary of the Invention

In one embodiment of the present invention are disclosed tubulin polymerization-inhibiting compounds of formula (I) 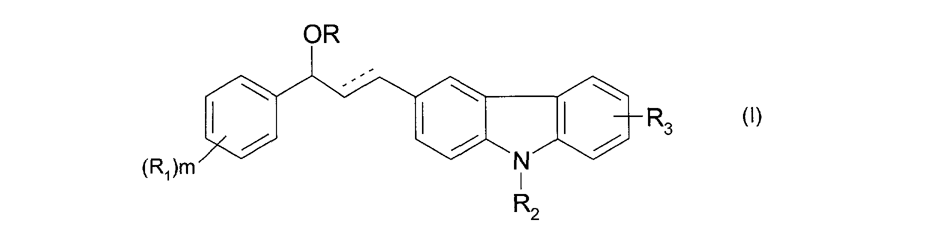 or a pharmaceutically acceptable salt, wherein
- R₁ is an alkoxy group
- R is selected from the group consisting of hydrogen or forms an oxo group
- R₂ is an alkyl group and
- R₃ is independently selected from the group consisting of hydrogen, halogen, alkyl, alkoxy, alkylsulfanyl, sulfoxide or sulfone and -NR₄R₅, wherein R₄ and R₅ are independently hydrogen or alkyl
- m is equal to 1, 2 or 3 and

In a preferred embodiment are disclosed compounds of formula (I) wherein the alkyl group contains 1 to 4 carbon atoms and more preferably 1 to 2 carbon atoms.

In another embodiment are disclosed compounds of formula (I) wherein R₁ is a methoxy group.

In a preferred embodiment, R₁ is methoxy, R₂ is an ethyl group and R₃ is hydrogen.

In still yet another embodiment is disclosed use of compounds of formula (I) for treating cancer diseases.

In still yet another embodiment is disclosed a first medical use for inhibiting tubulin polymerization in a mammal comprising administering to the mammal a therapeutically effective amount of formula (I).

In still yet another embodiment is disclosed a first medical use for treating cancer in a mammal comprising administering to the mammal a therapeutically effective amount of formula (I).

In still yet another embodiment is disclosed an antivascular compound of formula (I).

In still yet another embodiment is disclosed an antivascular method of treating cancer in a mammal in recognized need of such treatment comprising administering to the mammal a therapeutically effective amount of a compound of formula (I).

In still yet another embodiment is disclosed a first medical use for inhibiting tubulin polymerization in a mammal comprising administering to the mammal a therapeutically effective amount of formula (I) wherein R₁ is methoxy, R₂ is an ethyl group and R₃ is hydrogen.

In still yet another embodiment is disclosed a first medical use for treating cancer in a mammal comprising administering to the mammal a therapeutically effective amount of a 1-methoxyphenyl-3-(9-alkyl-9H-carbazol-3-yl)propan- or -2-propen-1-ol or 1-one.

In still yet another embodiment is disclosed a first medical use for inhibiting tubulin polymerization in a mammal comprising administering to the mammal a therapeutically effective amount of a 1-methoxyphenyl-3-(9-alkyl-9H-carbazol-3-yl)propan- or -2-propen-1-ol or 1-one.

In still yet another embodiment is disclosed an antivascular method of treating cancer in a mammal in recognized need of such treatment comprising administering to the mammal a therapeutically effective amount of a mono or 1-poly methoxyphenyl-3-(9-alkyl-9H-carbazol-3-yl)propan- or -2-propen-1-ol or 1-one.

In still yet another embodiment is disclosed a pharmaceutical composition which comprises a therapeutically effective amount of a compound of formula (I) in combination with a pharmaceutically acceptable carrier.

In still yet another embodiment is disclosed a pharmaceutical composition which comprises a therapeutically effective amount of a compound of formula (I), wherein R₁ is methoxy, R₂ is an ethyl group and R₃ is hydrogen in combination with a pharmaceutically acceptable carrier.

### Detailed Description of the Invention

### Definition of terms

The term "alkoxy", as used herein, refers to an alkyl group of one to 4 carbon atoms derived from a straight or branched hydrocarbon.

In still yet another embodiment is disclosed chemical compounds of formula (I) 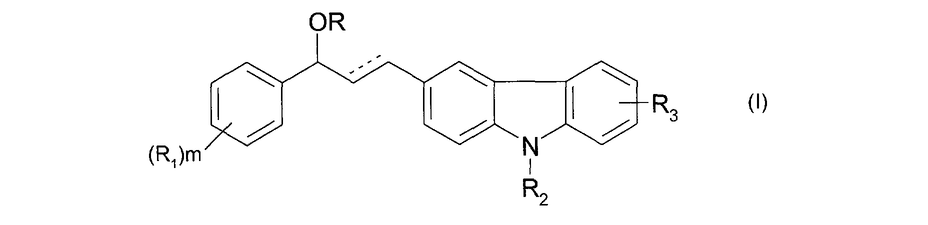 or a pharmaceutically acceptable salt, wherein
- R₁ is an alkoxy group containing 1 to 4 carbon atoms
- R is selected from the group consisting of hydrogen or forms an oxo group
- R₂ is an alkyl group and
- R₃ is independently selected from the group consisting of hydrogen, halogen, alkyl, alkoxy, alkylsulfanyl, sulfoxide or sulfone and -NR₄R₅, wherein R₄ and R₅ are independently hydrogen or alkyl
- m is equal to 1, 2 or 3

The term "alkyl" or "alkoxy" or "alkylsulfanyl" as used herein, refers to a group of one to 4 carbon atoms derived from a straight or branched chain hydrocarbon.

The term "pharmaceutically acceptable salt", as used herein, refers to salts, which are suitable for use in contact with the tissues of humans and lower animals. Pharmaceutically acceptable salts are described in detail in J. Pharmaceutical Sciences, 1977, 66:1 et seq. hereby incorporated by reference. Representative acid addition salts include acetate, citrate, aspartate, benzenesulfonate, hydrochloride, lactate, maleate, methanesulfonate, oxalate and phosphate.

### Methods of Synthesis

Compounds of the invention of general formula (I) are generally prepared according to the chemical routes described in Scheme 1

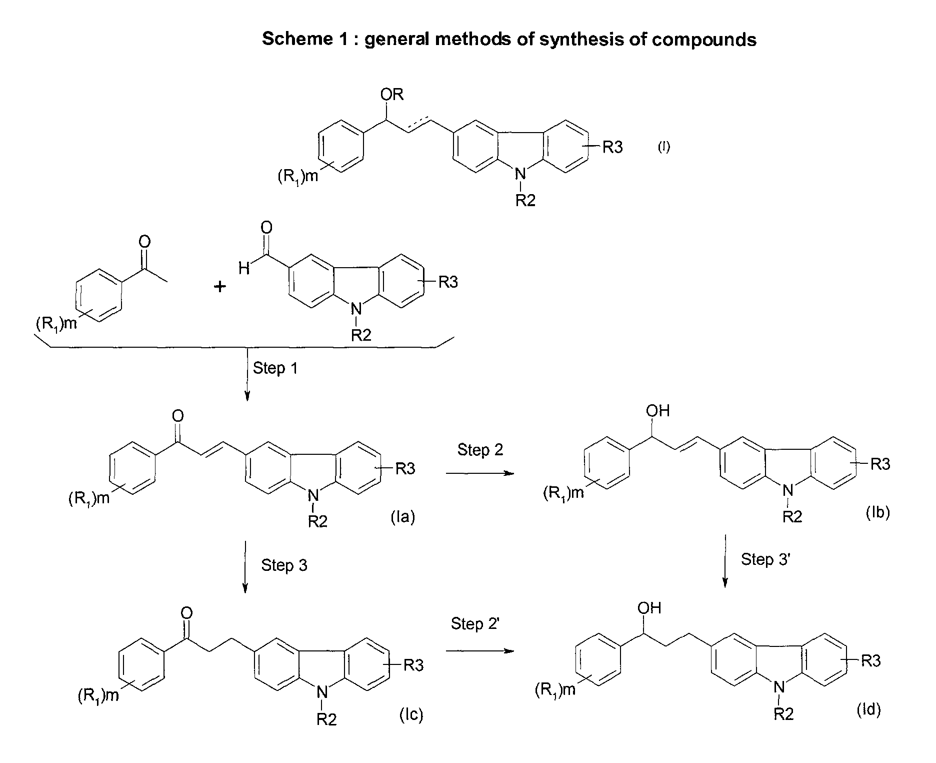

Compounds of general formula (Ia) are easily prepared by any method known by men of the art for the synthesis of chalcones. More specifically, according to Step 1 in Scheme 1, a convenient method is the condensation of an acetophenone, in which R₁ and m are as previously described, with a 3-carbazolecarboxaldehyde, in which R₂ and R₃ are as previously described, in the presence of a base. Usually the condensation is performed in a protic solvent such as alcohols using a mineral base such as an aqueous solution of sodium or potassium hydroxide. Starting acetophenones, in which R₁ and m are as previously described, and 3-carbazolecarboxaldehydes, in which R₂ and R₃ are as previously described, are either commercially available or prepared by already known methods.

Compounds of general formula (Ib) are easily prepared by any specific method of reduction of propenones to 2-propen-1-ols (Step 2 in Scheme 1) known by men of the art, using dedicated hydrides. More specifically usuful in that purpose are the methods of J. Cha (Bull. Korean Chem., 1998, 19, 236), G. Roos (Synlett, 1996, 1189) and R. Varma (Synth. Commun., 1985, 15, 985). The methods of G. Roos and R. Varma, that use sodium borohydride in methanol or in a mixture of methanol and tetrahydrofurane are specifically convenient.

Compounds of general formula (Ic) are easily prepared by any specific method of reduction of propenones to propanones (Step 3 in Scheme 1) known by men of the art, using either catalytic hydrogenation or dedicated hydrides. Catalytic hydrogenation is perforemed using either platinum, according for example to R. Li (J. Med. Chem. 1995, 38, 5031), or palladium, according for example to J. Corrie (Tetrahedron 1998, 54, 5407) or rhodium catalyst, according for example to R. Larsen (J. Org. Chem; 1996, 61, 3398). Among the various dedicated hydrides that can be used, the methods of E. Einholm (J. Org. Chem, 1995, 60, 4850), T. Kawakami (J. Org. Chem. 1996, 61, 376) and Y. Matsumoto (Synlett 1991, 349) are specifically convenient. Alternatively to hydrogen or hydrides, the reduction of propenones to propanones may be conveniently performed using Na₂S₂O₄ according to the method of J. Makrandi (Synth. Comm. 1990, 20, 1885).

Compounds of general formula (Id) are easily prepared either by reduction of propanones to propanonols (Step 2' in Scheme 1) or by reduction of 2-propen-1-ols to propanols (Step 3' in Scheme 2), using any of the common methods known by men of the art.

### Methods of Treatment

The present invention also provides pharmaceutical compositions, which comprise compounds of the present invention formulated together with one or more non-toxic pharmaceutically acceptable carriers. The pharmaceutical compositions may be specially formulated for oral administration in solid or liquid forms or for parenteral injection.

The term "parenteral", as used herein, refers to modes of administration, which include intravenous, intramuscular, intraperitoneal, subcutaneous and infusion.

Solid dosage forms for oral administration include capsules, tablets, pills, powders and granules. In such solid dosage forms, the active compound is mixed with at least one inert, pharmaceutically acceptable excipient or carrier.

Solid compositions of a similar type may also be employed as fillers in soft and hard-filled gelatin capsules.

The compounds of the present invention may be administered alone or mixed with other anticancer agents. Among the possible combinations, there may be mentioned
- alkylating agents and in particular cyclophosphamide, melphalan, ifosfamide, chlorambucil, busulfan, thiotepa, prednimustine, carmustine, lomustine, semustine, streptozotocin, decarbazine, temozolomide, procarbazine and hexamethylmelamine
- platinum derivatives such as in particular cisplatin, carboplatin or oxaliplatin
- antibiotic agents such as in particular bleomycin, mitomycin, dactinomycin,
- antimicrotubule agents such as in particular vinblastine, vincristine, vindesine, vinorelbine, taxoids (paclitaxel and docetaxel)
- anthracyclines such as in particular doxorubicin, daunorubicin, idarubicin, epirubicin, mitoxantrone, losoxantrone
- group I and II topoisomerases such as etoposide, teniposide, amsacrine, irinotecan, topotecan and tomudex,
- fluoropyrimidines such as 5-fluorouracil, UFT, floxuridine,
- cytidine analogues such as 5-azacytidine, cytarabine, gemcitabine, 6-mercaptomurine, 6-thioguanine
- adenosine analogues such as pentostatin, cytarabine or fludarabine phosphate
- methotrexate and folinic acid
- various enzymes and compounds such as L-asparaginase, hydroxyurea, trans-retinoic acid, suramine, dexrazoxane, amifostine, herceptin as well as oestrogenic and androgenic hormones.

It is also possible to combine a radiation treatment with the compounds of the present invention. This treatment may be administered simultaneously, separately or sequentially. The treatment will be adapted to the patient to be treated by the practitioner.

The invention will be more fully described by the following examples which must not be considered as a limitation of the invention.

### Example 1 : Preparation of 1-(2,4-dimethoxyphenyl)-3-(9-ethyl-9H-carbazol-3-yl)-propenone

To a 200 mL round bottom flask containing 50 mL of methanol, under stirring, is added, in succession, 3.6 g (0.020 mol) of 2',4'-dimethoxyacetophenone, 4.46 g (0.020 mol) of 9-ethyl-2-9H-carbazolecarboxaldehyde, and 2.5 mL of a 35 % aqueous solution of sodium hydroxide. The reaction flask is fitted with a reflux condenser and heated under reflux for 3 hours. At the end of this time, the reaction is allowed to cool and continued stirring at ambient temperature for an additional 84 hours. At the end of this time the solid that has formed is collected by suction filtration to give 5.88 g of a crude yellow solid. The solid is recrystallized from ethanol (600 mL) and collected by suction filtration. 3.60 g (49 % yield) of 1-(2,4-dimethoxy-phenyl)-3-(9-ethyl-9H-carbazol-3-yl)propenone is recovered. The product has the following characteristics :
- Mass Spectrum (EI-DCI) = 386 (M+)
- NMR Spectrum ¹H (300 MHz, (CD₃)₂SO d6, δ in ppm) : 1,35 (t, J = 7 Hz : 3H) ; 3,89 (s : 3H) ; 3,94 (s : 3H); 4,50 (q, J = 7 Hz : 2H) ; 6,57 (dd, J = 8,5 et 2 Hz : 1H) ; 6,74 (d, J = 2 Hz : 1H) ; 7,27 (t large, J = 7,5 Hz : 1H) ; 7,52 (t large, J = 8 Hz : 1H) ; 7,57 (d, J = 15,5 Hz : 1H) ; de 7,60 à 7,75 (mt : 3H) ; 7,76 (d, J = 15,5 Hz : 1H) ; 7,88 (dd, J = 8,5 et 1,5 Hz : 1H) ; 8,25 (d large, J = 8 Hz : 1H) ; 8,57 (s large : 1H).

### Example 2 : 1-(2,4-dimethoxyphenyl)-3-(9-ethyl-9H-carbazol-3-yl)propan-1-one

A 25 mL round bottom flask is charged with 200 mg (0.52 mmole) of 1-(2,4-dimethoxyphenyl)-3-(9-ethyl-9H-carbazol-3-yl)propenone which was prepared as above. The flask is then charged with 6.0 mL of cyclohexene, 4.0 mL of methanol and 200 mg of palladium, 10 wt. % on activated carbon. A stirr bar is added and the reaction flask is fitted with a reflux condenser and heated under reflux for 1.5 hours. At the end of this time, the reaction is allowed to cool and filtered through a short pad of celite. The crude product is then purified by flash column chromatography (diameter = 1 cm, volume of SiO₂ = 6 mL, eluant: cyclohexane /CH₂Cl₂ : 60 :40). 135.2 mg (67 % yield) of 1-(2,4-dimethoxyphenyl)-3-(9-ethyl-9H-carbazol-3-yl) propan-1-one was recovered. The product had the following characteristics :
- Mass Spectrum (EI-DCI) = 388 (M+)
- NMR Spectrum ¹H (300 MHz, (CD₃)₂SO d6, δ in ppm) : 1,32 (t, J = 7 Hz : 3H) ; 3,07 (t large, J = 7 Hz : 2H) ; 3,30 (t large, J = 7 Hz : 2H) ; 3,86 (s : 3H) ; 3,91 (s : 3H) ; 4,43 (q, J = 7 Hz : 2H) ; 6,63 (dd, J = 8,5 et 2,5 Hz : 1H) ; 6,68 (d, J = 2,5 Hz : 1H) ; 7,19 (t large, J = 7,5 Hz : 1H) ; 7,35 (dd, J = 8,5 et 2 Hz : 1H) ; 7,45 (t dédoublé, J = 8 et 1,5 Hz : 1H); 7,52 (d, J = 8,5 Hz : 1H); 7,59 (d, J = 8 Hz : 1H); 7,69 (d, J = 8,5 Hz : 1H) ; 8,01 (s large : 1H) ; 8,12 (d large, J = 8 Hz : 1H).

### Example 3 : Preparation of 1-(2,4-dimethoxyphenyl)-3-(9-ethyl-9H-carbazol-3-yl) propan-1-ol

A 25 mL round bottom flask is charged with 61 mg (0.15 mmole) of 1-(2,4-dimethoxyphenyl)-3-(9-ethyl-9H-carbazol-3-yl)propan-1-one that was prepared as described above. 5 mL methanol is added to form a suspension. The suspension is stirred. 1.5 mL of 1,4-dioxane is added to solublize the ketone. 40 mg (1.01 mmoles) of sodium borohydride is then added. After one hour, 2 mL of a saturated aqueous solution of NaCl is added to the solution. The contents of the flask are transferred to a separatory funnel and extracted with 10 mL of CH₂Cl₂. The CH₂Cl₂ layer is collected, dried over Na₂SO₄ and concentrated. The crude product is then purified by flash column chromatography (diameter = 1 cm, volume of SiO₂ = 6 mL, eluant: cyclohexane /CH₂Cl₂ : 70 :30). 51.1 mg (83 % yield) of 1-(2,4-dimethoxyphenyl)-3-(9-ethyl-9H-carbazol-3-yl)propan-1-ol. The product had the following characteristics
- Mass Spectrum (EI-DCI) = 390 (M+)
- NMR Spectrum ¹H (300 MHz, (CD₃)₂SO d6, δ in ppm): 1,31 (t, J = 7 Hz : 3H) ; de 1,80 à 2,10 (mt : 2H) ; de 2,70 à 2,95 (mt : 2H) ; 3,76 (s : 3H) ; 3,77 (s : 3H) ; 4,42 (q, J = 7 Hz : 2H) ; 4,89 (mt : 1H) ; 4,95 (d, J = 5 Hz : 1H) ; 6,54 (mt : 2H) ; 7,17 (t large, J = 7,5 Hz : 1H) ; 7,28 (dd, J = 8 et 1,5 Hz : 1H) ; 7,38 (d, J = 8 Hz : 1H) ; 7,45 (t, J = 8 et 1,5 Hz : 1H) ; 7,52 (d, J = 8 Hz : 1H) ; 7,59 (d, J = 8 Hz : 1H) ; 7,94 (s large : 1H) ; 8,13 (d large, J = 8 Hz : 1H).

### Example 4 : Preparation of 1-(2,5-dimethoxyphenyl)-3-(9-ethyl-9H-carbazol-3-yl) propenone

1-(2,5-dimethoxyphenyl)-3-(9-ethyl-9H-carbazol-3-yl)propenone is prepared according to example 1, starting with 2',5'-dimethoxyacetophenone in place of 2',4'-dimethoxyacetophenone.

### Example 5 : Preparation of 1-(3,4,5-trimethoxyphenyl)-3-(9-ethyl-9H-carbazol-3-yl) propenone

1-(3,4,5-trimethoxyphenyl)-3-(9-ethyl-9H-carbazol-3-yl)propenone is prepared according to example 1, starting with 3',4',5'-dimethoxyacetophenone in place of 2',4'-dimethoxyacetophenone.

### Example 6 : Preparation of 1-(2-methoxyphenyl)-3-(9-ethyl-9H-carbazol-3-yl) propenone

1-(2-methoxyphenyl)-3-(9-ethyl-9H-carbazol-3-yl)propenone is prepared according to example 1, starting with 2'-methoxyacetophenone in place of 2',4'-dimethoxyacetophenone.

### Example 7

Porcine brain tubulin was prepared by three cycles of polymerization-depolymerization (Shelanski *et al.*, 1973) followed by chromatography on phosphocellulose P11 (Whatman) (Weingarten *et al*., 1975). The eluted tubulin, depleted of MAPs (Microtubule Associated Proteins), was concentrated by ultra filtration, adjusted to 0.05 M MES, pH 6.8, 0.25 mM MgCl₂, 0.5 mM EGTA, 3.4 M glycerol and 0.2 mM GTP, and stored at -80°C at a concentration of 5-10 mg/ml. The tubulin concentration was measured by the method of Bradford using a 0.5 mg/ml bovine serum albumin solution as standard (Bradford, 1976).

Tubulin at 0-2°C was supplemented with 6 mM MgCl₂ and 1 mM GTP and used at a concentration of 10 µM (1 mg/ml). Compound of invention in DMSO (1 to 25 µM) were added to the tubulin solution. The samples (0.2 ml) were distributed in a pre cooled 96-well microplate and the plate was shacked for 10 minutes at room temperature. The polymerization was initiated by a temperature shift to 37°C.

The time-course of microtubule assembly was monitored turbidimetrically at 410 nm with a DYNATECH MR5000 microplate reader equipped with a thermostatically controlled plate holder. The temperature was set to 45°C giving a temperature inside each well of 37°C. The optical density (O.D.) was measured every 30 s during 30 minutes.

The kinetics of tubulin polymerization at various drug concentrations was superimposed. The lag time of the control curves ranged between 5 and 10 minutes. The lag time increased and the elongation rate decreased in the presence of drug. IC₅₀ is the concentration of drug for which the lag time was twice that of the control. This value was determined by visual inspection of the curves.

Results of IC₅₀ are mentioned in the following table.

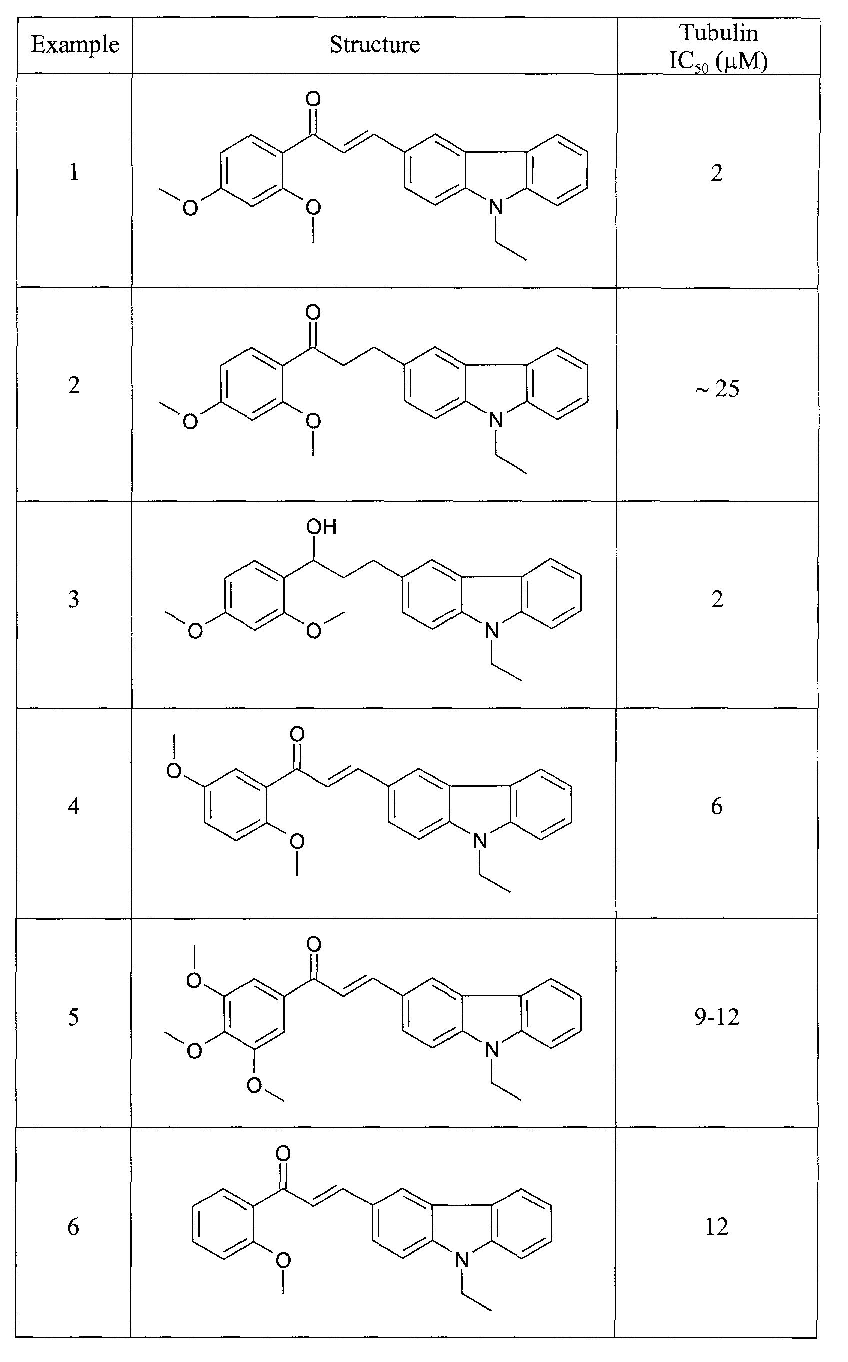

## Claims

**1.** Compounds of formula (I) 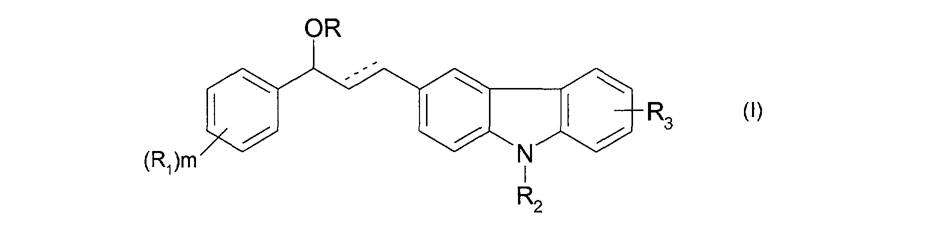 or a pharmaceutically acceptable salt, wherein
- R₁ is an alkoxy group containing 1 to 4 carbon atoms
- R is selected from the group consisting of hydrogen or forms an oxo group
- R₂ is an alkyl group and
- R₃ is independently selected from the group consisting of hydrogen, halogen, alkyl, alkoxy, alkylsulfanyl, sulfoxide, sulfonyl and -NR₄R₅, wherein R₄ and R₅ are independently hydrogen or alkyl
- m is equal to 1, 2 or 3

**2.** Compounds according to claim 1 wherein the alkyl group contains 1 to 4 carbon atoms and more preferably 1 to 2 carbon atoms.

**3.** Compounds according to claim 1 wherein R₁ is a methoxy group.

**4.** Compounds according to claim 1 wherein R₁ is methoxy, R₂ is an ethyl group and R₃ is hydrogen.

**6.** Use of compounds of formula (I) as a drug.

**7.** Use of product of formula (I) for inhibiting tubulin polymerization.

**8.** Use of compound of formula (I) for treating cancer.

**9.** Use of product of formula (I) as antivascular agent.

**10.** Use of product of formula (I) according to claim 7 wherein R₁ is methoxy, R₂ is an ethyl group and R₃ is hydrogen.

**11.** Use of mono or poly methoxyphenyl-3-(9-alkyl-9H-carbazol-3-yl)propan- or -2-propen-1-ol or -1-one according to claim 8 for treating cancer in a mammal in need of such treatment.

**12.** Use of mono or poly methoxyphenyl-3-(9-alkyl-9H-carbazol-3-yl)propan or -2-propen-1-ol or -1-one according to claim 7 for inhibiting tubulin polymerization in a mammal in recognized need of such treatment.

**13.** Pharmaceutical composition, which comprises a therapeutically effective amount of a compound of formula (I) in combination with a pharmaceutically acceptable carrier.

**14.** Pharmaceutical composition according to claim 13 which comprises a therapeutically effective amount of a mono or poly methoxyphenyl-3-(9-alkyl-9H-carbazol-3-yl)propan- or -2-propen--1-ol or -1-one, in combination with a pharmaceutically acceptable carrier.
